# EUROPEAN PATENT SPECIFICATION

(11) **EP 1 615 620 B1**
(45) Date of publication and mention of the grant of the patent: **01.02.2012**
(21) Application number: 04760104.2
(22) Date of filing: 21.04.2004
(51) Int. Cl.: A61K 9/00, G06T 7/00

(54) **METHOD OF MEASURING THE EFFICACY OF A SKIN TREATMENT PROGRAM**
VERFAHREN ZUR MESSUNG DER WIRKSAMKEIT EINES HAUTBEHANDLUNGSPROGRAMMS
PROCEDE DE MESURE DE L'EFFICACITE D'UN PROGRAMME THERAPEUTIQUE POUR LA PEAU

(30) Priority: 23.04.2003 US 421231
(43) Date of publication of application: 18.01.2006
(73) Proprietor: Johnson and Johnson Consumer Companies, Inc., Skillman, NJ 08558-9418 (US)
(72) Inventor: HUANG, Kelly, F-78100 Saint Germain en Laye (FR); KOLLIAS, Nikiforos, Skillman, NJ 08558 (US); KURTZ, Ellen, Ringoes, NJ 08551 (US); MARTIN, Katharine, Ringoes, NJ 08551 (US); NATTER, Florence, Hillsborough, NJ 08844 (US); PELLEGRINO, Arthur, Newtown, PA 18940 (US)
(74) Representative: Fisher, Adrian John
(86) International application number: PCT/US2004/012382
(87) International publication number: WO 2004/093844

(56) References cited:
- WO-A-02/37421
- US-A1- 2003 063 801
- MIYAMOTO AND HILLEBRAND: "The Beauty Imaging System: For the Objective Evaluation of Skin Condition" JOURNAL OF COSMETIC SCIENCE, SOCIETY OF COSMETIC CHEMISTS, NEW YORK, NY, US, vol. 53, 1 January 2001 (2001-01-01), pages 62-65, XP009101761 ISSN: 1525-7886
- AKAZAKI ET AL: 'Age-related changes in skin wrinkles assessed by a novel three-dimensional morphometric analysis' BRITISH JOURNAL OF DERMATOLOGY vol. 147, 2002, pages 689 - 695, XP002982192
- CORCUFF ET AL: 'The impact of aging on the microrelief of peri-orbital and leg skin' J. SOC. COSMET. CHEM. vol. 82, 1987, pages 145 - 152, XP002959951

## Description

### FIELD OF THE INVENTION

The present invention relates to a method of measuring the efficacy of a skin treatment program on human skin.

### BACKGROUND OF THE INVENTION

Humans are generally able to characterize a person to be within a particular age group based on an image of the person's face. This characterization may be accomplished by" various means, including the instrumental measurement of age-related changes in the skin , such as elasticity, cutaneous temperature, replicas, and water loss. Other techniques involve the clinical grading of photographs of human subjects by trained professionals to establish a correlation with skin aging. These trained professionals have been known to evaluate photographic images for wrinkling on the forehead, at the eye corners (crow's feet), in the cheekbone area, and at the mouth corners.

Computer imaging software is also known for artificially aging the image of an individual. For instance, such software is used by some Federal and state law enforcement agencies to age either a suspect or a victim when a current photograph of that person is not available. Computer aging has been particularly useful in cases involving missing children.

Computer imaging programs have also been useful in allowing a person to undergo a "virtual makeover", in which virtual "makeup" is applied to an image of a person or to a composite image created and displayed by the imaging program. In essence, this method involves superimposing a mask of various types and colors of makeup onto an image of a face.

Plastic surgeons have also used computer imaging to show a patient how his or her face might be altered as a result of plastic surgery.

These skin aging correlations and computer software programs, however, do not actually measure the effect of the use of a skin treatment program on a person's skin aging correlation. What is needed, then, is a method of measuring the efficacy of a skin treatment program on human skin to determine the constructive age change of the person's skin by the use of the product.
WO02/37421, US2003/063801 and Miyamoto & Hillebrand, Journal of Cosmetic Science, 53: 62-C5 (2001) disclose methods for measuring the efficacy of skin care products by comparing images of a subject's face before and after treatment

### SUMMARY OF THE INVENTION

In accordance with the present invention, there has been provided a method of measuring the efficacy of a skin treatment program on human skin, the method comprising:
a. accessing a multiplicity of photographic images of human faces, each image having associated with it a chronological age of the human whose likeness it captures;
b. assessing the multiplicity of photographic images of human faces for a facial parameter selected from eye corner wrinkles, nasolabial folds and under eye wrinkles by objective measurement by an instrument
c. scoring the facial parameter on each image according to a scoring system to create a parameter score for each image;
d. correlating the parameter scores to the chronological ages to obtain an age-parameter correlation;
e. scoring a photographie image of an untreated subject human face for the facial parameter to obtain a subject baseline parameter score Wherein the scoring is based on an assessment of the photographic mage of the intreated subject human face for the facial parameter and wherein this assessment is done by objective measurement by an instrument as on step (b) and scored according to the same scoring system used in step (c);
f. scoring a photographie image of subject human face for the facial parameter after treatment with a skin treatment program to obtain a subject treatment parameter score wherein the scoring is based on an assessment of the photographie image of the subject human face for the facial parameter and wherein this assessment is done by objective measurement by an instrument as a step (6) and scored according to the same storing system used a step;
g. determining the difference between the subject baseline parameter score and the subject treatment parameter score to obtain a treatment score difference; and
h. comparing the treatment score difference with the age-parameter correlation to determine a constructive age change of the human face effected by treatment of the human face with the skin treatment program.

### BRIEF DESCRIPTION OF THE FIGURES

Figure 1 is a flow chart overview of an embodiment of the method of the present invention.
Figure 2 is a graphical representation of the mean Parameter Score for each parameter in each age category.

### DETAILED DESCRIPTION OF THE INVENTION

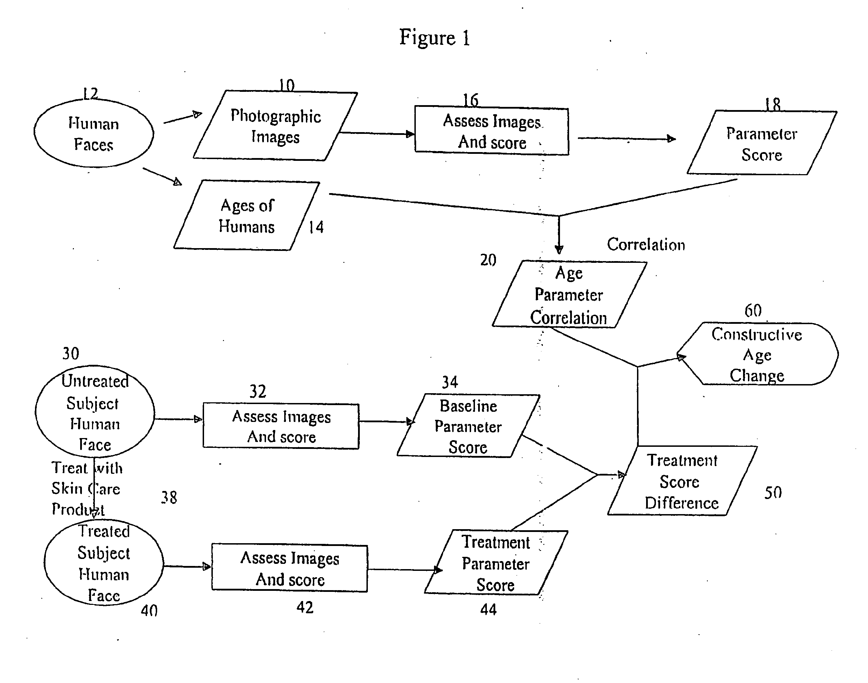

The present invention relates to a method of measuring the efficacy of a skin treatment program on human skin. More specifically, as shown in the embodiment of Figure 1, the present invention relates to first accessing a multiplicity of photographic images 10 of human faces 12, each image having associated with it a chronological age 14 of the human whose likeness it captures. Each of the multiplicity of photographic images is assessed 16 for a certain facial parameter (as specified below) and scored according to a scoring system to create a parameter score 18 for each image. The parameter scores of the images are then correlated to the chronological ages to obtain an age-parameter correlation 20. An untreated subject human face 30 is then scored 32 by the same scoring system for the facial parameter to obtain a subject baseline parameter score 34. The subject human face 40 is subsequently scored 42 by the same scoring system for the facial parameter after treatment with a skin care product 38 to obtain a subject treatment parameter score 44. The difference between the subject baseline parameter score and the subject treatment parameter score is determined to obtain a treatment score difference 50. The treatment score difference 50 is then compared with the age-parameter correlation 20 to determine a constructive age change 60 of the human face effected by treatment of the subject human face with the skin care product.

According to the present invention, a method is provided for measuring the efficacy of a skin treatment program on human skin, more specifically, the method provides a method for measuring the efficacy of an anti-aging skin treatment program in terms of the constructive age change, or apparent age change, of human skin after the use of the treatment program. As used herein, constructive age change means the apparent change in the age of a human due to changes in certain parameters of the human's skin that cause the human's skin to appear younger. The terminology "skin treatment program" refers to any skin care regimen that is intended to have a noticeable effect on human facial appearance and includes, but is not limited to nutritional programs, the use of topical skin care products, the use of skin care devices, oral dosage forms, massage therapy, radiation treatments, and the like and combinations thereof.

The method of the present invention involves accessing a multiplicity of photographic images of human faces with each image having associated with it a chronological age of the human whose likeness it captures. Photographic images include any conventional media capable of capturing the image of a human subject such as digital or analog electronic cameras as well as conventional film based photographic techniques. The images may be created with any type of light that is capable of capturing a chronological age related feature of a human subject, including but not limited to visible, infrared, ultra-violet, fluorescent, parallel polarized light and the like. The images may be captured and stored in electronic form or in the form of printed photographic images or in any other form in which the images may be visualized accurately. Preferably, the multiplicity of images are obtained from a multiplicity of humans of racial background and gender that are similar to the human subject being evaluated, and across a range of ages, preferably including ages that are less than and greater than the human subject. It is also preferable that the multiplicity of humans be of a sufficient number to provide statistical significance.

The images are then assessed for a facial parameter that changes as a human ages, selected from eye corner wrinkles (crow's feet), under-eye wrinkles and nasolabial folds, . The assessment of the facial parameters may be done by objective measurement, including measurement by an instrument, or by subjective measurement, such as by grading or scoring according to a predefined scale by a trained expert to obtain at least one parameter score for each image.

The parameter scores are then correlated to the chronological ages of the respective humans from which each image was obtained to obtain an age parameter correlation. This age parameter correlation provides information about which parameters exist with a certain frequency or severity in humans at particular chronological ages.

An untreated subject human face or an image of an untreated subject human face is scored for facial parameters according to the same method used to score the facial parameters of the multiplicity of photographic images to obtain a subject baseline parameter score. The subject is then treated with a skin treatment program, preferably with a skin care product, which may include without limitation, a lotion, a cream, a cleanser, a gel, a liquid, a powder, a toner, an astringent, and combinations thereof. Preferably, the skin care product is an anti-aging topical skin treatment product. After the subject skin has been treated with the product for a given length of time, the treated subject human face or an image of the treated subject human face is scored for facial parameters according to the same method used previously to score the untreated human face to obtain a subject treatment parameter score.

The difference between the subject treatment parameter score and the subject baseline parameter score is determined to obtain a treatment score difference. The treatment score difference is compared with the age parameter correlation to determine the constructive age change of the subject human face effected by the treatment of the human face with the skin treatment program.

The constructive age change of the subject human face is a measure of the efficacy of the skin treatment program on human skin and is useful in communicating the effectiveness of the skin treatment program to potential users of the product.

### EXAMPLE

### Image Assessment Study

Images (including visible and polarized images) of 421 Caucasian women ranging in age from 20-79 years were obtained. The images were obtained by taking a photograph using polarized light and a standard photograph of each subject. A "standard photograph", as used herein, refers to a photograph that is taken of the subject using visible light (e.g., light having a wavelength from about 400 to about 700 nm).

### Visible Light Photography

The standard photograph was obtained by illuminating the subject with two flash units that emitted visible light. The flash units were further equipped with a diffusing filter that was placed in front of each flash unit. A diffusing filter is a filter, which assists in uniformly dispersing light (e.g., to help eliminate "hot spots"). Examples of such diffusing filters include, but are not limited to, frosted glass filters such as a Broncolor Diffuser (Sinar Bron, Allschwil, Switzerland), metal grids which may be printed on glass substrates, or a diffusing reflective umbrella for indirect lighting.

The flash units were angled at the subject's skin to generate a gradient across the surface of the skin. Additionally, the flash units were mounted higher than the skin area of the subject and aimed at such skin area in order to give a gradient of light on the skin from the top to the bottom. The angle of the flash units was approximately 10 degrees from horizontal. This gradient visually enhanced various features of the skin such as the fine lines and wrinkles in the subject, e.g., the crow's feet around the eye and forehead or mouth area wrinkles.

### Polarized Light Photography.

A polarized photograph was also taken of each subject. A "polarized photograph", as used herein, refers to a photograph of the subject taken with a light source that emits light through a polarizing filter.

The camera and two flash units were on about the same plane as the subject's skin to be photographed, and the flash units were placed so that the angle formed by the flash units, the subject's skin, and the camera was about 45 degrees. A polarizing filter was placed in front of each flash unit. As used herein, a "polarizing filter" is a filter that filters incoming light to emit substantially only polarized light. As used herein, the term "substantially," means at least 75 percent, preferably 90 percent, and most preferably at least 95 percent.

Examples of a polarizing filter include, but are not limited to, polarizing plates such as those available from Edmund Scientific (Barrington, NJ USA), polarizing prisms such as Glan Thomson polarizing prisms, or a polarizing reflector that reflects light at about the Brewster angle.

A linear polarizing filter was used at the light source and the linear polarizing filter was arranged such that the electric field of the emitted light was about perpendicular to the plane formed by the light source, the person's skin, and the camera. The resulting image had a high degree of glare, which was further enhanced when an optical coupling medium, such as sebum or "oils," was present on the surface of the skin. The polarized image, thereby, allows an estimate to be made as to the oiliness of the subject's skin. It also provides insight into the number and severity of pores on the cheek and forehead areas of the facial skin. Other desired outcomes of polarized photography include, but are not limited to, an enhanced image of surface features such as fine lines, skin texture, scales and vellous hair.

The images were randomized and evaluated by a trained clinical grader for six age-related parameters: under-eye wrinkles, crow's feet, forehead wrinkles, frown lines, nasolabial folds, and cheek wrinkles. The grader was blinded to the chronological ages of the subjects of whom the images were taken. The grader scored each parameter on a 10-point ordinal scale with the following ratings: 0-none; 1-3=mild; 4-6=moderate; 7-9=severe. The objective of this portion of the study was to correlate the parameter score variables with age.

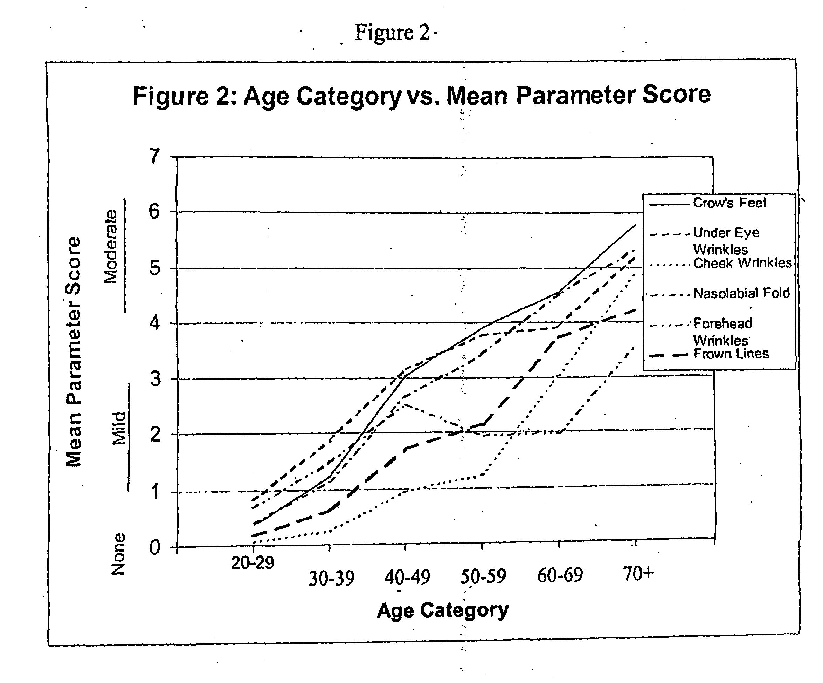

Figure 2 is a graphical representation of the mean parameter score for each parameter in each age category.

The Mantel-Haenszel correlation statistic tests the null hypothesis of no association against the alternative of linear association in at least one age stratum. A p-value of 0.05 was used to declare statistical significance, and SAS version 8.2 statistical software (Cary, NC) was used for all statistical analyses.

Among the 421 women whose images were scored, the mean age was 38.1 years (standard deviation (SD) 13.4, median 35.0), and ranged from 20 to 79 years. The percentage of women aged 20-29, 30-39, 4-49, 50-59, 60-69, and ≥70 years was 31%, 28%, 21%, 13%, 6%, and 2% respectively.

Table 1 presents the number and percentage of women with ratings of none, mild, moderate, and severe for each decade of age. For each parameter, the distribution of rating shifts towards greater severity corresponding to greater age. The p-value was statistically significant for each of the parameters (P<0.0001), indicating a strong linear association of age and parameter scores.

**Table 1**

| | 20-29 Yr. | 30-39 Yr. | 40-49 Yr. | 50-59 Yr. | 60-89 Yr. | 70+Yr. |
|---|---|---|---|---|---|---|
| Crow's Feet | 0.38 | 1.22 | 3.05 | 3.89 | 4.56 | 5,78 |
| Under Eye Wrinkles | 0.82 | 1.87 | 3.17 | 3.77 | 3.92 | 5.22 |
| Cheek Wrinkles | 0.06 | 0.25 | 0.95 | 1.23 | 3.04 | 4.89 |
| Nasolabial Fold | 0.42 | 1.14 | 2.67 | 3.42 | 4.52 | 5.33 |
| Forehead Wrinkles | 0.89 | 1.47 | 2.53 | 1.94 | 1.96 | 3.56 |
| Frown Lines | 0.17 | 0.61 | 1.71 | 2.15 | 3.72 | 4.22 |

As seen in Table 2, using Spearman correlation coefficient, age was positively correlated with each of the parameters, and was greatest for crow's feet (r=0.791), under-eye wrinkles (r=0.774) and nasolabial folds (r=0.774) . The pairwise correlations among the parameters indicated positive associations also.

**Table 2: Spearman Correlation**

| | Age | Crow's Feet | Under-Eye Wrinkles | Cheek Wrinkles | Nasolabial Folds | Forehead Wrinkles | Frown Lines |
|---|---|---|---|---|---|---|---|
| Age | 1.000 | 0.791 | 0.774 | 0.544 | 0.774 | 0,429 | 0.629 |
| Crow's Feet | | 1.000 | 0.840 | 0.564 | 0.775 | 0.472 | 0.583 |
| Under-Eye Wrinkles | | | 1.000 | 0.569 | 0.720 | 0.505 | 0.610 |
| Cheek Wrinkles | | | | 1.000 | 0.536 | 0.314 | 0.522 |
| Nasolabial Folds | | | | | 1.000 | 0.424 | 0.523 |
| Forehead Wrinkles | | | | | | 1.000 | 0.335 |
| Frown Lines | | | | | | | 1.000 |

### Treatment Study

Twenty five women subjects participated in a randomized, placebo-controlled, single-center, within-subject comparison treatment study. The women who were selected for the study had moderate to severe photodamaged facial skin. A trained clinical grader rated each of the women for facial parameters at the beginning of the study or baseline to determine a baseline parameter score, after 1 month, after 2 months, and after 3 months to determine a treatment parameter score. The parameters that were rated were under-eye wrinkles, crow's feet, nasolabial folds, and cheek wrinkles. The rating system involved using the same trained clinical grader as in the grading of the images described hereinbefore, and involved using the same scoring scale as that used to determine the age parameter correlation as described above.

During the study, each woman subject treated the skin on one half of her face with a skin care product formulation for treating aging parameters and the skin on the other half of her face with a placebo formulation. Each subject treated her facial skin in this way twice daily for the duration of the study, using the same formulation on the same half of her face. Neither the subjects nor the grader knew which formulation was the skin care product formulation or which was the placebo formulation, or in other words, the study was double-blind.

The composition of the formulation was as follows.

| **Ingredient** | **CTFA name** | **Function** | **%wt/wt** |
|---|---|---|---|
| **Lipid Phase** | | | |
| Arlacel 165 | Glyceryl Stearate & PEG-100 Stearate | emulsifier | 3.8000 |
| Brij 76 | Steareth-10 | emulsifier | 1.4000 |
| Procol ST 20G | Ceteareth 20 and Stearyl Alcohol | emulsifier | 3.0000 |
| Procol CS 20D | Ceteareth 20 and Cetearyl Alcohol | emulsifier | 3.0000 |
| Wickenol 171 | Octyl Hydroxystearate | emollient | 5.8000 |
| Tween 80 | Polysorbate 80 | emulsifier | 0.7000 |
| Stearyl Alcohol | Stearyl alcohol | emollient | 0.5000 |
| Vitamin A alcohol blend | Retinol 50C in Polysorbate 20 | skin conditioner | 0.2700 |
| BHT | Butylated hydroxytoluene | antioxidant | 0.1000 |
| | | | |
| **Water Phase** | | | |
| DI Water | Water | vehicle | 64.1700 |
| Sepitonic M3 | Mg Aspartate & Zn gluconate & Cu Gluconate | Skin conditioner | 1.0000 |
| Disodium EDTA | Disodium EDTA | chelator | 0.1000 |
| Glycerin | Glycerin | humectant | 4.0000 |
| Keltrol CG-BT | Xanthan Gum | Thickener | 0.3000 |
| Methylparaben | Methylparaben | preservative | 0.2500 |
| Propylparaben | Propylparaben | preservative | 0.1500 |
| | | | |
| **Post Additions** | | | |
| Emeressence 1160 | Phenoxyethanol | preservative | 0.7300 |
| Dimethicone 47V | Dimethicone, 100 cst | skin conditioner | 2.5000 |
| Actiquench | butylene glycol. water, camellia oleifera extract | antioxidant | 0.1000 |
| Calcium D-Pantothenate | Calcium D-Pantothenate | antioxidant | 0.0500 |
| Erythrobic Acid | Isoascorbic Acid | antioxidant | 0.0500 |
| Simulgel NS | Hydroxyethyl acrylate/Sodium acryloyldimethyltaurate and squalane and polysorbate 60 | thickener | 1.5000 |
| DMAE | Dimethylaminoethanol | skin conditioner | 2.5000 |
| | | | |
| **Buffer Premix** | | | |
| DI water | DI water | solvent | 1.5800 |
| Malic Acid | Malic Acid | pH adjuster | 1.0100 |
| Glypure | Glycolic Acid, 70% | pH adjuster | 1.4400 |
| | | | |
| **TOTAL** | | | 100.0000 |

The formulation was prepared according to the following process:

| **Batch Process** |
|---|
| ***Oil* Phase** |
| 1. Heat kettle to 70°C +/- 10°C and add Arlacel, Brij, Procols, Stearyl Alcohol. Mix until uniform. |
| 2. Cool to 65°C +/- 5°C, add BHT, Wickenol, and Tween 80. Hold at 65°C +/- 5°C for phasing. |
| 3. Blanket lipid tank with argon, turn on yellow lights and add retinol just prior to phasing |
| ***Water Phase*** |
| 4. Add Water to beaker. Bubble argon through until dissolved oxygen < 2ppm. |
| 5. Blanket water phase with argon. |
| 6. Premix glycerin and xanthan and add to batch, mix well and begin heating. Add EDTA and Sepitonic M3. |
| 7. At 80°C +/- 5°C, add Methyl and Propyl paraben. Mix until dissolved. |
| 8. Cool to 65°C +/- 5°C and hold for phasing. |
| ***Phasing*** |
| 9. Add Oil phase (60°C - 70°C) to water phase (60°C - 70°C) with rapid mixing. Maintain argon blanket. |
| ***Finishing*** |
| 8. At 65°C or below (45°C -65°C) Add simulgel and mix until uniform. Begin cooling batch. |
| 9. Below 50°C, and Emeressence, Dimethicone, Actiquench, Calcium D-Pantothenate, and Erythorbic acid. |
| 10. Below 40°C, add DMAE, adjust pH to 6 (5.6 - 6.3) with water/malic/glypure premix. |
| 11. QS with water. |

The composition of the placebo formulation was the same as that of the skin care formulation, except that the dimethylaminoethanol (DMAE) and the retinol were omitted.

The estimated change in parameter score for each 10-year change in age in the subset of women aged 38 to 57 was computed for the image assessment study of the 421 women described above. These parameter score changes were compared to parameter score changes observed among the women in the product study. These changes were defined as 10 times the mean change of the product study divided by the 10-year change in the parameter score from the image assessment study, which provided the proportionate reduction in years, or constructive age reduction. As shown in Table 3, the constructive age reduction following active treatment with the skin care product for 3 months was 13.2 years (95% Confidence Interval: 7.3 to 19.3) for under-eye wrinkles, 7.2 years (95% Confidence Interval: 4.5 to 10.0) for crow's feet, 0.8 years (95% Confidence Interval: 0.3 year increase to 2.0 year decrease) for nasolabial folds, and 12.3 years (95% Confidence Interval: 7.2 to 15.2) for cheek wrinkles.

**Table 3**

| Parameter | Mean Change | 95%. CI | | 10-yr. Change | Constructive Age Reduction | 95% CI | |
|---|---|---|---|---|---|---|---|
| | | Lower | Upper | | | Lower | Upper |
| Under-eye wrinkles | -0.60 | -0.33 | -0.87 | 0.45 | 13.2 years | 7.3 | 19.3 |
| Crow's feet | -0.68 | -0.42 | -0.94 | 0.94 | 7.2 years | 4.5 | 10.0 |
| Nasolabial Fold | -0.08 | +0.03 | -0.19 | 0.97 | 0.8 Years | -0.3 | 2.0 |
| Cheek Wrinkles | -1.08 | -0.82 | -1.34 | 0.88 | 12.3 years | 7.2 | 15.2 |

The specification and example above are presented to aid in the complete and non-limiting understanding of the invention disclosed herein. Since many variations and embodiments of the invention can be made without departing from its scope, the invention resides in the claims hereinafter appended.

## Claims

1. A method of measuring the efficacy of a skin treatment program on human skin, the method comprising:
(a) accessing a multiplicity of photographic images (10) of human faces (12), each image having associated with it a chronological age (14) of the human whose likeness it captures;
(b) assessing the multiplicity of photographic images of human faces for a facial parameter selected from eye corner wrinkles, nasolabial folds and under eye wrinkles, by objective measurement by an instrument;
(c) scoring the facial parameter on each image according to a scoring system to create a parameter score (18) for each image;
(d) correlating the parameter scores to the chronological ages to obtain an age-parameter correlation (20);
(e) scoring a photographic image of an untreated subject human face (30) for the facial parameter to obtain a subject baseline parameter score (34), wherein the scoring is based on an assessment of the photographic image of the untreated subject human face for the facial parameter and wherein this assessment is done by objective measurement by an instrument as in step (b) and scored according to the same scoring system used in step (c);
(f) scoring a photographic image of the subject human face (40) for the facial parameter after treatment with a skin treatment program, to obtain a subject treatment parameter score (44), wherein the scoring is based on an assessment of the photographic image of the subject human face for the facial parameter and wherein this assessment is done by objective measurement by an instrument as in step (b) and scored according to the same scoring system used in step (c);
(g) determining the difference between the subject baseline parameter score and the subject treatment parameter score to obtain a treatment score difference (50); and
(h) comparing the treatment score difference with the age-parameter correlation to determine a constructive age change (60) of the human face effected by treatment of the human face with the skin treatment program.

2. The method of claim 1 wherein the facial parameter is under eye wrinkles.

3. The method of claim 1 wherein the facial parameter is nasolabial folds.

4. The method of claim 1 wherein the facial parameter is eye corner wrinkles.

## Patentansprüche

1. Verfahren zum Messen der Wirksamkeit eines Hautbehandlungsprogramms auf menschlicher Haut, wobei das Verfahren Folgendes umfasst:
(a) Zugreifen auf mehrere photographische Bilder (10) menschlicher Gesichter (12), wobei jedem Bild ein chronologisches Alter (14) des Menschen, dessen Bildnis es darstellt, zugeordnet ist;
(b) Einschätzen der mehreren photographischen Bilder menschlicher Gesichter hinsichtlich eines Gesichtsparameters, der aus Augenwinkelfältchen, Nasen-Lippen-Furche und Unteraugenfältchen gewählt ist, durch objektive Messung durch ein Instrument;
(c) Bewerten des Gesichtsparameters in jedem Bild in Übereinstimmung mit einem Bewertungssystem, um eine Parameterbewertung (18) für jedes Bild zu erzeugen;
(d) Korrelieren der Parameterbewertungen mit den chronologischen Altern, um eine Alter-Parameter-Korrelation (20) zu erhalten;
(e) Bewerten eines photographischen Bildes eines nicht behandelten menschlichen Gesichts (30) einer Person im Hinblick auf den Gesichtsparameter, um eine Personengrundlinien-Parameterbewertung (34) zu erhalten, wobei die Bewertung auf einer Einschätzung des photographischen Bildes des nicht behandelten menschlichen Gesichts einer Person hinsichtlich des Gesichtsparameters beruht und wobei diese Einschätzung durch objektive Messung durch ein Instrument wie im Schritt (b) erfolgt und gemäß demselben Bewertungssystem, wie es im Schritt (c) verwendet wird, bewertet wird;
(f) Bewerten eines photographischen Bildes des menschlichen Gesichts (40) der Person hinsichtlich des Gesichtsparameters nach der Behandlung mit einem Hautbehandlungsprogramm, um eine Behandlungsparameterbewertung (44) der Person zu erhalten, wobei die Bewertung auf einer Einschätzung des photographischen Bildes des menschlichen Gesichts der Person hinsichtlich des Gesichtsparameters beruht und wobei diese Einschätzung durch objektive Messung durch ein Instrument wie im Schritt (b) erfolgt und gemäß demselben Bewertungssystem, wie es im Schritt (c) verwendet wird, bewertet wird;
(g) Bestimmen der Differenz zwischen der Grundlinienparameterbewertung der Person und der Behandlungsparameterbewertung der Person, um eine Behandlungsbewertungsdifferenz (50) zu erhalten; und
(h) Vergleichen der Behandlungsbewertungsdifferenz mit der Alter-Parameter-Korrelation, um eine konstruktive Altersänderung (60) des menschlichen Gesichts, die durch Behandlung des menschlichen Gesichts mit dem Hautbehandlungsprogramm bewirkt wird, zu bestimmen.

2. Verfahren nach Anspruch 1, wobei sich der Gesichtsparameter auf die Unteraugenfältchen bezieht.

3. Verfahren nach Anspruch 1, wobei sich der Gesichtsparameter auf die Nasen-Lippen-Furche bezieht.

4. Verfahren nach Anspruch 1, wobei sich der Gesichtsparameter auf die Augenwinkelfältchen bezieht.

## Revendications

1. Procédé de mesure de l'efficacité d'un programme de traitement de la peau sur une peau humaine, le procédé comportant les étapes consistant à :
(a) accéder à une multiplicité d'images photographiques (10) de visages humains (12), chaque image se voyant affecter un âge chronologique (14) de l'humain dont elle illustre le portrait ;
(b) évaluer la multiplicité d'images photographiques de visages humains pour obtenir un paramètre facial choisi parmi les rides aux coins des yeux, les plis nasolabiaux et les rides sous les yeux, par une mesure objective au moyen d'un instrument ;
(c) noter le paramètre facial sur chaque image selon un système de notation pour créer un score (18) de paramètre pour chaque image ;
(d) corréler les scores de paramètre aux âges chronologiques pour obtenir une corrélation âge-paramètre (20) ;
(e) noter une image photographique du visage (30) d'un sujet humain non traité en termes de paramètre facial pour obtenir un score (34) de paramètre de référence du sujet, la notation étant basée sur une évaluation de l'image photographique du visage du sujet humain non traité en termes de paramètre facial et ladite évaluation étant effectuée par une mesure objective au moyen d'un instrument comme dans l'étape (b) et notée selon le même système de notation utilisé à l'étape (c) ;
(f) noter une image photographique du visage (40) du sujet humain en termes de paramètre facial après traitement par un programme de traitement de la peau, pour obtenir un score (44) de paramètre de traitement du sujet, la notation étant basée sur une évaluation de l'image photographique du visage du sujet humain en termes de paramètre facial et ladite évaluation étant effectuée par une mesure objective au moyen d'un instrument comme dans l'étape (b) et notée selon le même système de notation utilisé à l'étape (c) ;
(g) déterminer l'écart entre le score de paramètre de référence du sujet et le score de paramètre de traitement du sujet pour obtenir un écart (50) de scores de traitement ; et
(h) comparer l'écart de scores de traitement à la corrélation âge-paramètre pour déterminer une variation constructive (60) d'âge du visage humain causé par le traitement du visage humain au moyen du programme de traitement de la peau.

2. Procédé selon la revendication 1, le paramètre facial étant les rides sous les yeux.

3. Procédé selon la revendication 1, le paramètre facial étant les plis nasolabiaux.

4. Procédé selon la revendication 1, le paramètre facial étant les rides aux coins des yeux.
